(19)

Europäisches Patentamt
European Patent Office
Office européen des brevets

(11) **EP 4 674 443 A2**

(12) **EUROPEAN PATENT APPLICATION**

(43) Date of publication:
**07.01.2026 Bulletin 2026/02**

(21) Application number: **25217719.1**

(22) Date of filing: **21.01.2021**

(51) International Patent Classification (IPC):
***A61L 24/10*** *(2006.01)*

(52) Cooperative Patent Classification (CPC):
**A61L 24/0031; A61L 24/108; C07K 7/08;**
A61L 2430/36; Y02P 20/141

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.02.2020 JP 2020032544**

(62) Document number(s) of the earlier application(s) in
accordance with Art. 76 EPC:
**21759966.1 / 4 112 089**

(71) Applicants:
- **MENICON CO., LTD.
Naka-ku
Nagoya-shi, Aichi 460-0006 (JP)**
- **The University of Osaka
Osaka 565-0871 (JP)**

(72) Inventors:
- **UESUGI, Koji
Kasugai-shi, 487-0032 (JP)**
- **EGUCHI, Hidetoshi
Suita-shi, 565-0871 (JP)**
- **GOTOH, Kunihito
Suita-shi, 565-0871 (JP)**

(74) Representative: **Winter, Brandl - Partnerschaft
mbB
Alois-Steinecker-Straße 22
85354 Freising (DE)**

Remarks:
- The complete document including Reference Table(s) and the Sequence Listing(s) can be downloaded from the EPO website
- This application was filed on 21-11-2025 as a divisional application to the application mentioned under INID code 62.

(54) **MATERIAL FOR PREVENTING LEAKAGE OF DIGESTIVE LIQUID FROM DIGESTIVE ORGAN AND MATERIAL FOR PROTECTING ORGAN FROM DIGESTION BY DIGESTIVE LIQUID**

(57) The present invention provides a self-assembling peptide gel for use in preventing a leak of digestive fluid and a self-assembling peptide gel for use in protecting an organ against digestion by a digestive fluid. The self-assembling peptide gel comprises a self-assembling peptide and water, whereby the self-assembling peptide gel has an adhesion persistence ratio with respect to a collagen sheet of at least 40% and a decomposition ratio of 35% or less in pancreatic juice treatment at 37°C for 7 days, and the self-assembling peptide gel is positively charged as a whole.

Fig.1

EP 4 674 443 A2

## Description

Technical Field

The present invention relates to a digestive fluid leak preventative material and an organ protective material against digestion by a digestive fluid.

Background Art

In an operation on a digestive organ, a digestive fluid sometimes leaks from the digestive organ postoperatively. For example, with regard to a pancreas, which produces pancreatic juice containing enzymes that decompose proteins, fats, and sugars, the pancreatic juice sometimes leaks from the pancreas after an operation. This is called a pancreatic fistula, and the pancreatic fistula is regarded as an important problem among postoperative complications because the pancreatic fistula may induce an inflammation by collapsing the pancreas itself and surrounding tissues like digestion.

As a typical operation on the pancreas, there are given a pancreaticoduodenectomy, which is performed for, for example, a tumor near a pancreatic head, and a distal pancreatectomy, which is performed for, for example, a tumor in a pancreatic body or a pancreatic tail. In the pancreaticoduodenectomy, the pancreatic head, a bile duct, a duodenum, or part of a stomach is resected, and the pancreas, the bile duct, and the stomach are each sutured to a small intestine. In addition, in the distal pancreatectomy, the tail part of the pancreatic body or the pancreatic tail is resected/automatically sutured with a linear stapler, or a stump is closed with a suture. At this time, fibrin glue is sometimes applied to an anastomosed portion and/or the stump for the purpose of preventing a leak of pancreatic juice due to a suture failure at the anastomosed portion and/or a closure failure at the stump. However, the fibrin glue may be digested by activated pancreatic juice, and hence is not satisfactory from the viewpoint of preventing a pancreatic juice leak.

In addition, in each of the above-mentioned operative methods, a drain is inserted near the anastomosed portion or the pancreatic stump, and the presence or absence of occurrence of a pancreatic fistula is judged on the basis of, for example, an amylase value in a fluid discharged through the drain and duration thereof in days. When the occurrence of a pancreatic fistula is recognized, a treatment, such as medication or discharge of pancreatic juice out of the body with the drain, is performed. In addition, for example, in Patent Literature 1, there is a proposal of a method of occluding a pancreatic fistula through use of a self-assembling peptide. However, the method is intended to occlude a pancreatic fistula by closing a hole after drain removal through use of a self-assembling peptide.

Citation List

Patent Literature

[PTL 1] JP 2019-508175 A

Summary of Invention

Technical Problem

The present invention has been made in order to solve the problem of the related art described above, and a primary object of the present invention is to provide a digestive fluid leak preventative material capable of exhibiting a satisfactory effect from the viewpoint of preventing a pancreatic juice leak. More particularly, the present invention is to provide a self-assembling peptide gel for use in preventing a leak of digestive fluid and a self-assembling peptide gel for use in protecting an organ against digestion by a digestive fluid.

Solution to Problem

According to an aspect of the present invention, there is provided a digestive fluid leak preventative material, including a self-assembling peptide gel containing: a self-assembling peptide; and water, having an adhesion persistence ratio with respect to a collagen sheet of at least 40%, and having a decomposition ratio of 35% or less in pancreatic juice treatment at 37°C for 7 days.

According to the present invention, there is provided a self-assembling peptide gel for use in preventing a leak of digestive fluid, wherein the self-assembling peptide gel comprises a self-assembling peptide, and water, whereby the self-assembling peptide gel has an adhesion persistence ratio with respect to a collagen sheet of at least 40 % and a decomposition ratio of 35 % or less in pancreatic juice treatment at 37°C for 7 days, and the self-assembling peptide gel formed using the self-assembling peptide having a positive charge at a physiological pH is positively charged as a whole.

In one embodiment, the self-assembling peptide includes a self-assembling peptide having a positive charge at a physiological pH.

In one embodiment, the self-assembling peptide includes a self-assembling peptide having a charge of from +1 to +4 at the physiological pH.

In one embodiment, the digestive fluid leak preventative material has a viscosity of 3.0 Pa·s or more, which is measured under a temperature condition of 25°C using a rotational viscometer at a rotational speed of 0.5 rpm.

In one embodiment, the self-assembling peptide includes a self-assembling peptide containing the following amino acid sequence (A):

$$\text{Amino acid sequence (A):} \qquad a_1b_1c_1b_2a_2b_3db_4a_3b_5c_2b_6a_4$$

in the amino acid sequence, $a_1$ to $a_4$ each represent a basic amino acid residue; $b_1$ to $b_6$ each represent an uncharged polar amino acid residue and/or a hydrophobic amino acid residue, provided that at least five thereof represent hydrophobic amino acid residues; $c_1$ and $c_2$ each represent an acidic amino acid residue; and "d" represents a hydrophobic amino acid residue or an uncharged polar amino acid residue.

In one embodiment, the self-assembling peptide includes at least one kind selected from self-assembling peptides set forth in SEQ ID NOS: 1 to 13 and 16 to 18.

In one embodiment, the digestive fluid leak preventative material is for use in preventing a leak of pancreatic juice from a pancreas.

According to another aspect of the present invention, there is provided a method of preventing a leak of a digestive fluid from a digestive organ, the method including applying the digestive fluid leak preventative material to a digestive organ in need of prevention of a leak of a digestive fluid.

According to still another aspect of the present invention, there is provided an organ protective material against digestion by a digestive fluid, including a self-assembling peptide gel containing: a self-assembling peptide; and water, having an adhesion persistence ratio with respect to a collagen sheet of at least 40%, and having a decomposition ratio of 35% or less in pancreatic juice treatment at 37°C for 7 days.

According to another aspect of the present invention, there is provided a self-assembling peptide gel for use in protecting an organ against digestion by a digestive fluid, wherein the self-assembling peptide gel comprises a self-assembling peptide and water, whereby the self-assembling peptide gel has an adhesion persistence ratio with respect to a collagen sheet of at least 40 % and a decomposition ratio of 35 % or less in pancreatic juice treatment at 37°C for 7 days, and the self-assembling peptide gel formed using the self-assembling peptide having a positive charge at a physiological pH is positively charged as a whole.

In one embodiment, the self-assembling peptide includes a self-assembling peptide having a positive charge at a physiological pH.

In one embodiment, the self-assembling peptide includes a self-assembling peptide having a charge of from +1 to +4 at the physiological pH.

In one embodiment, the organ protective material has a viscosity of 3.0 Pa·s or more, which is measured under a temperature condition of 25°C using a rotational viscometer at a rotational speed of 0.5 rpm.

In one embodiment, the self-assembling peptide includes a self-assembling peptide containing the following amino acid sequence (A):

$$\text{Amino acid sequence (A):} \qquad a_1b_1c_1b_2a_2b_3db_4a_3b_5c_2b_6a_4$$

in the amino acid sequence, $a_1$ to $a_4$ each represent a basic amino acid residue; $b_1$ to $b_6$ each represent an uncharged polar amino acid residue and/or a hydrophobic amino acid residue, provided that at least five thereof represent hydrophobic amino acid residues; $c_1$ and $c_2$ each represent an acidic amino acid residue; and "d" represents a hydrophobic amino acid residue or an uncharged polar amino acid residue.

In one embodiment, the self-assembling peptide includes at least one kind selected from self-assembling peptides set forth in SEQ ID NOS: 1 to 13 and 16 to 18.

In one embodiment, wherein the organ protective material is for use in protecting an organ against digestion by pancreatic juice.

According to still another aspect of the present invention, there is provided a method of protecting an organ against digestion by a digestive fluid, the method including applying the organ protective material to a surface of an organ to be protected.

The invention relates to the following aspects:

According to a first aspect, a digestive fluid leak preventative material comprises a self-assembling peptide gel containing a self-assembling peptide and water. This material has an adhesion persistence ratio with respect to a collagen sheet of at least 40%, and has a decomposition ratio of 35% or less in pancreatic juice treatment at 37°C for 7 days.

According to a second aspect, which depends on the first aspect, the self-assembling peptide includes a self-assembling peptide, which has a positive charge at a physiological pH.

According to a third aspect, which depends on the second aspect, the self-assembling peptide includes a self-assembling peptide having a charge of from +1 to +4 at the physiological pH.

According to a fourth aspect, which depends on any one of the first to third aspects, the digestive fluid leak preventative material has a viscosity of 3.0 Pa·s or more, which is measured under a temperature condition of 25°C using a rotational viscometer at a rotational speed of 0.5 rpm.

According to a fifth aspect, which depends on any one of the first to fourth aspects, the self-assembling peptide includes a self-assembling peptide containing the following amino acid sequence (A):

Amino acid sequence (A):    $a_1b_1c_1b_2a_2b_3db_4a_3b_5c_2b_6a_4$

in the amino acid sequence, $a_1$ to $a_4$ each represent a basic amino acid residue; $b_1$ to $b_6$ each represent an uncharged polar amino acid residue and/or a hydrophobic amino acid residue, provided that at least five thereof represent hydrophobic amino acid residues; $c_1$ and $c_2$ each represent an acidic amino acid residue; and "d" represents a hydrophobic amino acid residue or an uncharged polar amino acid residue.

According to a sixth aspect, which depends on any one of the first to fifth aspects, the self-assembling peptide includes at least one kind selected from self-assembling peptides set forth in SEQ ID NOS: 1 to 13 and 16 to 18.

According to a seventh aspect, which depends on any one of the first to sixth aspects, the digestive fluid leak preventative material is for use in preventing a leak of pancreatic juice from a pancreas.

According to an eighth aspect, a method of preventing a leak of a digestive fluid from a digestive organ comprises applying the digestive fluid leak preventative material of any one of the first to seventh aspects to a digestive organ in need of prevention of a leak of a digestive fluid.

According to a ninth aspect, an organ protective material against digestion by a digestive fluid comprises a self-assembling peptide gel containing a self-assembling peptide and water. This material has an adhesion persistence ratio with respect to a collagen sheet of at least 40%, and has a decomposition ratio of 35% or less in pancreatic juice treatment at 37°C for 7 days.

According to a tenth aspect, which depends on the ninth aspect, the self-assembling peptide includes a self-assembling peptide, which has a positive charge at a physiological pH.

According to an eleventh aspect, which depends on the tenth aspect, the self-assembling peptide includes a self-assembling peptide, which has a charge of from +1 to +4 at the physiological pH.

According to a twelfth aspect, which depends on any one of the ninth to eleventh aspects, the organ protective material has a viscosity of 3.0 Pa·s or more, which is measured under a temperature condition of 25°C using a rotational viscometer at a rotational speed of 0.5 rpm.

According to a thirteenth aspect, which depends on any one of the ninth to twelfth aspects, the self-assembling peptide includes a self-assembling peptide, which contains the following amino acid sequence (A):

Amino acid sequence (A):    $a_1b_1c_1b_2a_2b_3db_4a_3b_5c_2b_6a_4$

in the amino acid sequence, $a_1$ to $a_4$ each represent a basic amino acid residue; $b_1$ to $b_6$ each represent an uncharged polar amino acid residue and/or a hydrophobic amino acid residue, provided that at least five thereof represent hydrophobic amino acid residues; $c_1$ and $c_2$ each represent an acidic amino acid residue; and "d" represents a hydrophobic amino acid residue or an uncharged polar amino acid residue.

According to a fourteenth aspect, which depends on any one of the ninth to thirteenth aspects, the self-assembling peptide includes at least one kind selected from self-assembling peptides set forth in SEQ ID NOS: 1 to 13 and 16 to 18.

According to a fifteenth aspect, which depends on any one of the ninth to fourteenth aspects, the organ protective material is for use in protecting an organ against digestion by pancreatic juice.

According to a sixteenth aspect, a method of protecting an organ against digestion by a digestive fluid comprises applying the organ protective material of any one of the ninth to fifteenth aspects to a surface of an organ to be protected.

Advantageous Effects of Invention

According to the present invention, the material capable of exhibiting a satisfactory effect from the viewpoint of preventing a pancreatic juice leak from the pancreas can be provided by using the self-assembling peptide gel having an adhesion persistence ratio with respect to the collagen sheet equal to or higher than a predetermined value and having a decomposition ratio equal to or lower than a predetermined value in the pancreatic juice treatment.

Brief Description of Drawings

FIG. 1 is a graph showing the adhesion persistence ratios of self-assembling peptide gels.

FIG. 2 is a graph showing amylase values in ascitic fluids from rats.

FIG. 3 includes microscopic observation photographs of resection sites on the pancreas of rats after 3 days from an operation.

Description of Embodiments

Embodiments of the present invention are described below. However, the present invention is not limited to the embodiments.

A. Definitions of Terms

(1) As used herein, the term "self-assembling peptide" means a peptide capable of spontaneously assembling through an interaction between peptide molecules in an aqueous solution to form a fibrous assembly having a nanometer-sized width (hereinafter referred to as "nanofiber"). The interaction between peptide molecules is not particularly limited, and encompasses non-covalent interactions, for example, electrostatic interactions such as a hydrogen bond, an interionic interaction, and a van der Waals force, and a hydrophobic interaction. The formation of the nanofiber may be recognized through, for example, electron microscopic observation.

(2) As used herein, the term "gel" means a viscoelastic substance having both viscous properties and elastic properties. In one embodiment, a substance having a viscosity of 3 Pa·s or more, which is measured under the temperature condition of 25°C using a rotational viscometer at a rotational speed of 0.5 rpm, may be called a gel.

(3) As used herein, the term "self-assembling peptide gel" means a product obtained when nanofibers formed through spontaneous assembling of a self-assembling peptide form a three-dimensional network structure and hold water molecules therein to gelate.

(4) As used herein, the term "hydrophilic amino acid" encompasses: basic amino acids, such as arginine (Arg/R), lysine (Lys/K), and histidine (His/H); acidic amino acids, such as aspartic acid (Asp/D) and glutamic acid (Glu/E); and uncharged polar amino acids, such as tyrosine (Tyr/Y), serine (Ser/S), threonine (Thr/T), asparagine (Asn/N), glutamine (Gln/Q), and cysteine (Cys/C). The alphabetical letters in each set of parentheses in the foregoing are the three-letter representation and one-letter representation of the amino acid.

(5) As used herein, the term "hydrophobic amino acid" encompasses non-polar amino acids, such as alanine (Ala/A), leucine (Leu/L), isoleucine (Ile/I), valine (Val/V), methionine (Met/M), phenylalanine (Phe/F), tryptophan (Trp/W), glycine (Gly/G), and proline (Pro/P). The alphabetical letters in each set of parentheses in the foregoing are the three-letter representation and one-letter representation of the amino acid.

B. Digestive Fluid Leak Preventative Material

A digestive fluid leak preventative material according to an embodiment of the present invention is composed of a self-assembling peptide gel containing: a self-assembling peptide; and water, has an adhesion persistence ratio with respect to a collagen sheet of 40% or more, and has a decomposition ratio of 35% or less in pancreatic juice treatment at 37°C for 7 days. The digestive fluid leak preventative material can stably adhere so as to cover a sutured portion and/or a closed portion of a digestive organ without being significantly digested (decomposed) by a digestive fluid, and hence can suitably prevent a leak of the digestive fluid from the digestive organ. In addition, the digestive fluid leak preventative material promotes cell growth at its adhesion site, and thus can promote the cure of the leaking portion (sutured portion and/or closed portion). Herein, the prevention of a leak of a digestive fluid is a concept including the prophylaxis of a leak of a digestive fluid.

Examples of the digestive organ include a stomach, a duodenum, a small intestine, a liver, a gallbladder, a pancreas, and a spleen. In one embodiment, a leak of pancreatic juice from the pancreas and/or a leak of bile from the gallbladder (bile duct) may be prevented.

The adhesion persistence ratio of the digestive fluid leak preventative material with respect to a collagen sheet is typically 40% or more, preferably 45% or more, more preferably 50% or more. The upper limit of the adhesion persistence ratio is not particularly limited, but may be set to, for example, 80%, or for example, 70%. When the adhesion persistence ratio falls within the above-mentioned ranges, the digestive fluid leak preventative material can stably keep adhering so as to cover the sutured portion and/or the closed portion of the digestive organ. The adhesion persistence ratio may be measured by a method described in Examples. In addition, the adhesion persistence ratio may be improved by, for example, using a self-assembling peptide having a positive charge at a physiological pH (pH=7.4), increasing a peptide concentration, or increasing a viscosity.

The decomposition ratio of the digestive fluid leak preventative material in pancreatic juice treatment (exposure to pancreatic juice) at 37°C for 7 days is typically 35% or less, preferably 30% or less, more preferably 15% or less, still more preferably from 0% to 10%. When the decomposition ratio in the pancreatic juice treatment falls within the above-mentioned ranges, the digestive fluid leak preventative material can stably keep adhering to the surface of the digestive organ without being significantly decomposed by a digestive fluid to be prevented from leaking. Herein, the decomposition ratio in the pancreatic juice treatment may be measured, as described in Examples, by using simulated pancreatic juice (e.g., a 1 mg/mL aqueous solution of a digestive enzyme preparation containing 1 g of Japanese Pharmacopoeia pancreatin in 1 g thereof). A peptide is generally decomposed by pancreatic juice containing a peptidase, but a self-assembled peptide assembly (specifically a self-assembling peptide gel) is hardly decomposed by pancreatic juice, and hence can suitably function as a digestive fluid leak preventative material.

The viscosity of the digestive fluid leak preventative material may be, for example, 3.0 Pa·s or more, preferably 9.0 Pa·s or more, more preferably 15.0 Pa·s or more, still more preferably 25.0 Pa·s or more, even more preferably 35.0 Pa·s or more. In addition, the viscosity of the digestive fluid leak preventative material may be, for example, 1,000 Pa·s or less, or for example, 100 Pa·s or less. The viscosity is a viscosity measured under the temperature condition of 25°C using a rotational viscometer at a rotational speed of 0.5 rpm.

The pH of the digestive fluid leak preventative material is preferably a pH at which the self-assembling peptide included in the self-assembling peptide gel can maintain its charge at the physiological pH. The pH of the digestive fluid leak preventative material may be, for example, from 5.0 to 9.0, preferably from 5.5 to 8.0, more preferably from 5.5 to 7.5.

As described above, the digestive fluid leak preventative material is composed of the self-assembling peptide gel containing the self-assembling peptide and water. The self-assembling peptide gel may further contain a pharmaceutically acceptable additive component in accordance with a purpose.

B-1. Self-assembling Peptide

A self-assembling peptide having a positive charge at the physiological pH (pH=7.4) may be preferably used as the self-assembling peptide. Specifically, there may be used a self-assembling peptide having a charge of, for example, from +1 to +4, preferably from +1 to +3, more preferably +2 or +3, still more preferably +2 per molecule at the physiological pH. Such self-assembling peptide can suitably form a nanofiber and a gel. In addition, a self-assembling peptide gel formed by such self-assembling peptide can exhibit such a reversible self-assembling ability that its fluidity is increased by a physical stimulus, such as stirring, vibration, or the application of a shear force, and the fluidity is decreased by stopping the physical stimulus. Accordingly, there is an advantage in that a burden in administration using an elongate instrument, such as a syringe or a catheter, is reduced. Further, the self-assembling peptide gel formed using the self-assembling peptide having a positive charge can be excellent in retentivity on the surface of an organ such as a digestive organ, or of a mucous membrane. The reason why such effect is exhibited is presumably as follows, though the present invention is not limited thereto: the self-assembling peptide gel formed using the self-assembling peptide having a positive charge is positively charged as a whole and causes an electrostatic interaction with a negatively charged cell surface, with the result that an adhesive force therebetween can be improved.

Meanwhile, a self-assembling peptide gel formed by a self-assembling peptide having no charge at the physiological pH or a self-assembling peptide having a negative charge thereat (consequently a self-assembling peptide gel that is uncharged or negatively charged as a whole) cannot provide an electrostatic attraction between itself and a negatively charged cell surface, and hence its retentivity on the surface of an organ such as a digestive organ, or of a mucous membrane may be insufficient.

In addition, the self-assembling peptide having no charge at the physiological pH typically has an irreversible self-assembling ability, and starts the formation of a nanofiber through a change in pH of a peptide solution or a reaction with an inorganic salt to gelate. Accordingly, the gelation of such self-assembling peptide proceeds after its administration in a solution state to the sutured portion and/or the closed portion of the digestive organ, and hence the self-assembling peptide spreads in such a manner as to follow the surface of the administration site until its gelation, with the result that it is difficult to quickly form a gel having a sufficient thickness in some cases.

The charge of the self-assembling peptide means the sum total of the charges of the amino acid residues contained in the molecule of the peptide. The charge at the physiological pH may be calculated using, for example, a program available on the website of PROTEIN CALCULATOR v3.4 (http://protcalc.sourceforge.net/).

The number of amino acid residues constituting the self-assembling peptide is, for example, 9 or more, preferably from 10 to 40, more preferably from 10 to 32, still more preferably from 12 to 32. The N-terminal amino group and/or the C-terminal carboxyl group of the peptide may be appropriately protected with a protecting group, such as an acetyl group or an amide group.

As the self-assembling peptide, there may be used, for example, a peptide in which: amino acid residues at odd-numbered positions (or even-numbered positions) are alternately arranged acidic amino acid residues and basic amino acid residues; of the acidic amino acid residues, any appropriate one to four, preferably two or three amino acid residues are each substituted by an uncharged polar amino acid residue or a hydrophobic amino acid residue; and all amino acid residues at even-numbered positions (or odd-numbered positions) are hydrophobic amino acid residues. In an aqueous solution, such peptide forms a β-sheet formed of a hydrophobic surface in which only hydrophobic amino acid residues are arranged and a hydrophilic surface including hydrophilic amino acid residues, and two β-sheets extend in a state of overlapping each other with their hydrophobic surfaces on the inside, with the result that nanofibers can be formed. The nanofibers can further assemble through an interaction between the hydrophilic surfaces to form a network structure, to thereby cause gelation. The formation of a β-sheet structure may be recognized by, for example, measuring a molar ellipticity by a circular dichroism measurement method and confirming that a molar ellipticity at 216 nm takes a negative value. In addition, the formation may also be recognized by using FT-IR analysis to detect a peak attributable to a β-sheet, which appears around 1,620 $cm^{-1}$, or a peak attributable to an antiparallel β-sheet, which appears around 1,690 $cm^{-1}$.

The self-assembling peptide may be exemplified by a peptide containing the following amino acid sequence (A). The self-assembling peptide may be a peptide formed of the following amino acid sequence (A), or may be a peptide in which some, for example, one to five of any appropriate amino acid residues are added to each of the N-terminus and/or the C-terminus thereof as long as the effect of the present invention is obtained. In addition, as required, the N-terminal amino group of the peptide may be acetylated, and the C-terminal carboxyl group thereof may be amidated.

Amino acid sequence (A):    $a_1 b_1 c_1 b_2 a_2 b_3 d b_4 a_3 b_5 c_2 b_6 a_4$

In the amino acid sequence, $a_1$ to $a_4$ each represent a basic amino acid residue; $b_1$ to $b_6$ each represent an uncharged polar amino acid residue and/or a hydrophobic amino acid residue, provided that at least five thereof represent hydrophobic amino acid residues; $c_1$ and $c_2$ each represent an acidic amino acid residue; and "d" represents a hydrophobic amino acid residue or an uncharged polar amino acid residue.

In one embodiment, in the amino acid sequence, all of $b_1$ to $b_6$ represent hydrophobic amino acid residues. $b_1$ to $b_6$ may each independently represent an alanine residue, a valine residue, a leucine residue, or an isoleucine residue, preferably an alanine residue or a leucine residue, and it is more preferred that all of $b_1$ to $b_6$ represent leucine residues, or that five thereof represent leucine residues and one thereof represent an alanine residue.

In one embodiment, in the amino acid sequence, "d" represents an alanine residue, a leucine residue, an asparagine residue, a serine residue, or a glutamine residue.

In one embodiment, in the amino acid sequence, all of $a_1$ to $a_4$ represent arginine residue or lysine residue, preferably arginine residue.

In one embodiment, in the amino acid sequence, all of $c_1$ and $c_2$ represent aspartic acid residue or glutamic acid residue, preferably aspartic acid residue.

The self-assembling peptide that may be used in the present invention is not limited to the peptide containing the amino acid sequence (A). For example, a peptide described in WO 2007/000979 A1 may be applied.

Specific examples of the self-assembling peptide include peptides set forth in SEQ ID NOS: 1 to 13 and 16 to 18. Of those, peptides set forth in SEQ ID NOS: 1 to 4 are more preferred. The self-assembling peptides may be used alone or in combination thereof to the extent that the effect of the present invention is obtained.

Table 1

| Amino acid sequence | Charge at pH 7.4 | SEQ ID NO: |
|---|---|---|
| n-RLDLRLALRLDLR-c | +2 | 1 |
| n-RLDLRLSLRLDLR-c | +2 | 2 |
| n-RLALRLDLRLDLR-c | +2 | 3 |
| n-KRLDLNLRLDLRK-c | +3 | 4 |
| n-RLDLRLLLRLDLR-c | +2 | 5 |

(continued)

| Amino acid sequence | Charge at pH 7.4 | SEQ ID NO: |
|---|---|---|
| n-RADLRLALRLDLR-c | +2 | 6 |
| n-RLDLRLALRLDAR-c | +2 | 7 |
| n-RADLRLLLRLDLR-c | +2 | 8 |
| n-RADLRLLLRLDAR-c | +2 | 9 |
| n-RLDLRALLRLDLR-c | +2 | 10 |
| n-RLDLRLLARLDLR-c | +2 | 11 |
| n-RLNLRLDLRLNL-c | +2 | 12 |
| n-RAQARAQARAQARAQA-c | +4 | 13 |
| n-RASARASARASARASA-c | +4 | 16 |
| n-RASARASARASARADA-c | +3 | 17 |
| n-RASARADARADARADA-c | +1 | 18 |

The blending ratio of the self-assembling peptide in the digestive fluid leak preventative material (self-assembling peptide gel) may be appropriately set in accordance with, for example, a desired adhesion persistence ratio and a desired decomposition ratio after the pancreatic juice treatment. The blending ratio may be, for example, from 0.3 w/w% to 6.0 w/w%, preferably from 0.5 w/w% to 5.0 w/w%, more preferably from 0.6 w/w% to 4.0 w/w%, still more preferably from 0.8 w/w% to 3.0 w/w%, even more preferably from 1.0 w/w% to 2.0 w/w%.

The self-assembling peptide may be prepared by any appropriate method, such as a liquid-phase synthesis method, a solid-phase synthesis method, or a molecular biological technique. The self-assembling peptide may be in the form of a salt due to its production method, and a digestive fluid leak preventative material using the self-assembling peptide in the form of a salt is also encompassed in the embodiments of the present invention.

B-2. Water

Examples of the water include distilled water, deionized water, and pure water. The water may form an aqueous medium, such as physiological saline, buffered saline (e.g., PBS), a phosphate buffer, or an isotonic aqueous buffer, together with the additive component to be described later.

The blending ratio of the water in the digestive fluid leak preventative material (self-assembling peptide gel) is, for example, 80 wt% or more, preferably 85 wt% or more, more preferably 90 wt% or more.

B-3. Additive Component

Any appropriate additive may be selected by a person skilled in the art as the pharmaceutically acceptable additive in accordance with a purpose and the like. Specific examples thereof include a pH adjuster, a tonicity agent, a drug, a preservative, an excipient, a stabilizer, a filler, and a solubilizing agent. The additives may be used alone or in combination thereof.

Examples of the pH adjuster include citric acid, trisodium citrate, succinic acid, monosodium succinate, disodium succinate, sodium acetate, sodium hydrogen carbonate, sodium carbonate, phosphoric acid, disodium hydrogen phosphate, sodium dihydrogen phosphate, histidine, and lysine.

Examples of the tonicity agent include sodium chloride, polyethylene glycol, dextran, mannitol, sorbitol, inositol, glucose, fructose, lactose, xylose, mannose, maltose, sucrose, trehalose, and raffinose.

Any appropriate drug may be used as the drug as long as the effect of the present invention is obtained. Specific examples of the drug include an anti-inflammatory agent, a protease inhibitor (e.g., gabexate mesilate), a steroid, and an antibiotic.

B-4. Production Method for Digestive Fluid Leak Preventative Material

The digestive fluid leak preventative material may be produced by any appropriate production method. For example, the digestive fluid leak preventative material may be obtained by mixing the self-assembling peptide, water, and the additive as an option. As required, each of the components before mixing or their mixture may be sterilized. Any appropriate method that may be applied in the art may be used as each of a mixing method and a sterilization method. The self-assembling peptide gel composing the digestive fluid leak preventative material is stable even under a high-temperature state, and hence may be subjected to high-pressure steam sterilization.

## C. Method of Preventing Leak of Digestive Fluid

According to another aspect of the present invention, there is provided a method of preventing a leak of a digestive fluid. A method of preventing a leak of a digestive fluid according to an embodiment of the present invention includes applying the digestive fluid leak preventative material described in the section B to a digestive organ in need of prevention of a leak of a digestive fluid.

An application method may involve, for example, applying the digestive fluid leak preventative material to the surface of the digestive organ so as to cover or close a site at which a digestive fluid may leak (e.g., a suture failure site, a perforation, or a cut surface). In one embodiment, the digestive fluid leak preventative material is ejected onto the target site using a tubular instrument, such as a syringe, a needle, a pipette, a catheter, or a tube, and is spread with a spatula, a swab, or the like as required. In addition, for example, the digestive fluid leak preventative material may be applied by being sprayed using a spray. As described above, the digestive fluid leak preventative material is capable of being increased in fluidity by a physical stimulus such as the application of a shear force, and returning to its original fluidity by stopping the physical stimulus. Accordingly, when the tubular instrument is used, while the digestive fluid leak preventative material can be easily ejected with a small pressing force by increasing its fluidity, the digestive fluid leak preventative material can be caused to stably adhere to the surface of the digestive organ by being quickly returned to its original fluidity after the ejection.

An application amount may be any appropriate amount as long as the effect of the present invention is obtained. The application amount may be, for example, such an amount as to achieve an application thickness of 0.01 mm or more and less than 3 cm, preferably 0.05 mm or more and less than 1 cm.

Examples of the digestive organ in need of prevention of a leak of a digestive fluid include a stomach, a duodenum, a small intestine, a liver, a gallbladder, a pancreas, and a spleen. In particular, the digestive fluid leak preventative material and the method of preventing a leak of a digestive fluid may be preferably applied to a duodenum, a gallbladder, or a pancreas, and are effective for the prevention of a pancreatic fistula and/or a biliary fistula. The digestive organ is preferably a digestive organ of a human or a non-human animal (e.g., a non-human primate, a dog, a cat, a rat, a bovine, a pig, or a sheep).

## D. Organ Protective Material

According to another aspect of the present invention, there is provided an organ protective material. An organ protective material according to an embodiment of the present invention is composed of a self-assembling peptide gel containing: a self-assembling peptide; and water, has an adhesion persistence ratio with respect to a collagen sheet of 40% or more, and has a decomposition ratio of 35% or less in pancreatic juice treatment at 37°C for 7 days. The organ protective material can prevent a peripheral organ from being significantly digested (decomposed) by a digestive fluid even when a leak of the digestive fluid occurs.

The organ to be protected is an organ present in an abdominal cavity (viscus), and examples thereof include: internal organs, such as a stomach, a duodenum, a small intestine, a large intestine, a liver, a gallbladder, a pancreas, a spleen, and a kidney; and circulatory organs such as a blood vessel.

The adhesion persistence ratio of the organ protective material with respect to a collagen sheet is typically 40% or more, preferably 45% or more, more preferably 50% or more. The upper limit of the adhesion persistence ratio is not particularly limited, but may be set to, for example, 80%, or for example, 70%. When the adhesion persistence ratio falls within the above-mentioned ranges, the organ protective material can stably keep adhering to the surface of the organ to be protected. The adhesion persistence ratio may be measured by a method described in Examples. In addition, the adhesion persistence ratio may be improved by, for example, using a peptide having a positive charge at a physiological pH (pH=7.4), increasing a peptide concentration, or increasing a viscosity.

The decomposition ratio of the organ protective material in pancreatic juice treatment at 37°C for 7 days is typically 35% or less, preferably 30% or less, more preferably 15% or less, still more preferably from 0% to 10%. When the decomposition ratio in the pancreatic juice treatment falls within the above-mentioned ranges, the organ protective material can prevent the organ to be protected from being brought into contact with a digestive fluid, without being significantly decomposed by the digestive fluid. Herein, the decomposition ratio in the pancreatic juice treatment may be measured, as described in Examples, by using simulated pancreatic juice (e.g., a 1 mg/mL aqueous solution of a digestive enzyme preparation containing 1 g of Japanese Pharmacopoeia pancreatin in 1 g thereof). A peptide is generally decomposed by pancreatic juice containing a peptidase, but a self-assembled peptide assembly (specifically a self-assembling peptide gel) is hardly decomposed by pancreatic juice, and hence can suitably function as an organ protective material.

The viscosity of the organ protective material may be, for example, 3.0 Pa·s or more, preferably 9.0 Pa·s or more, more preferably 15.0 Pa·s or more, still more preferably 25.0 Pa·s or more, even more preferably 35.0 Pa·s or more. In addition, the viscosity of the organ protective material may be, for example, 1,000 Pa·s or less, or for example, 100 Pa·s or less. The viscosity is a viscosity measured under the temperature condition of 25°C using a rotational viscometer at a rotational speed of 0.5 rpm.

The pH of the organ protective material is preferably a pH at which the self-assembling peptide included in the self-assembling peptide gel can maintain its charge at the physiological pH. The pH of the organ protective material may be, for example, from 5.0 to 9.0, preferably from 5.5 to 8.0, more preferably from 5.5 to 7.5.

As described above, the organ protective material is composed of the self-assembling peptide gel containing the self-assembling peptide and water. The self-assembling peptide gel may further contain a pharmaceutically acceptable additive component in accordance with a purpose. As the self-assembling peptide gel, the same self-assembling peptide gel as that composing the digestive fluid leak preventative material described in the section B may be used.

E. Method of protecting Organ

According to another aspect of the present invention, there is provided a method of protecting an organ against digestion by a digestive fluid. A method of protecting an organ according to an embodiment of the present invention includes applying the organ protective material described in the section D to a surface of an organ to be protected.

An application method may involve, for example, applying the organ protective material to the surface of the organ to be protected so as to cover at least part of the surface of the organ. In one embodiment, the organ protective material is ejected onto the target site using a tubular instrument, such as a syringe, a needle, a pipette, a catheter, or a tube, and is spread with a spatula, a swab, or the like as required. In addition, for example, the organ protective material may be applied by being sprayed using a spray. As described above, the organ protective material is capable of being increased in fluidity by a physical stimulus such as the application of a shear force, and returning to its original fluidity by stopping the physical stimulus. Accordingly, when the tubular instrument is used, while the organ protective material can be easily ejected with a small pressing force by increasing its fluidity, the organ protective material can be caused to stably adhere to the surface of the organ by being quickly returned to its original fluidity after the ejection.

An application amount may be any appropriate amount as long as the effect of the present invention is obtained. The application amount may be, for example, such an amount as to achieve an application thickness of 0.01 mm or more and less than 3 cm, preferably 0.05 mm or more and less than 1 cm.

Examples of the organ in need of protection against digestion by a digestive fluid include: internal organs, such as a stomach, a duodenum, a small intestine, a large intestine, a liver, a gallbladder, a pancreas, a spleen, and a kidney; and circulatory organs such as a blood vessel. The organ is preferably an organ of a human or a non-human animal (e.g., a non-human primate, a dog, a cat, a rat, a bovine, a pig, or a sheep) .

Examples

Now, the present invention is more specifically described by way of Examples. However, the present invention is not limited to these Examples.

<<Viscosity>>

The viscosity (Pa·s) of a sample was measured using a rotational viscometer (manufactured by Toki Sangyo Co., Ltd., product name: "TVE-20LT") as a viscosity measurement apparatus. Specifically, the measurement was performed as described below. First, about 0.2 g of the sample was weighed in a sample cup, which was mounted onto the main body of the viscometer, and water controlled to a temperature of 25±0.2°C was circulated for 15 minutes or more. After that, within Measurement Range M, a cone rotor (manufactured by Toki Sangyo Co., Ltd., 3°×R9.7) was rotated at 0.5 rpm, and the average of viscosities displayed on the screen about 15 minutes, about 20 minutes, and about 25 minutes after the start of the rotation was adopted as the viscosity.

<<Decomposition Ratio in Pancreatic Juice Treatment>>

200 μL of simulated pancreatic juice (1 mg/mL aqueous solution of a product manufactured by Maruishi Pharmaceutical Co., Ltd. under the product name Pancreatin Bulk Powder "Maruishi" (digestive enzyme preparation containing 1 g of Japanese Pharmacopoeia pancreatin in 1 g thereof)) or PBS was left to stand still on 0.1 g of a sample (gel) placed in a 1.5 mL tube at 37°C for 7 days. To the tube after the standing still, 1 mL of trifluoroacetic acid (manufactured by Wako Pure Chemical Industries, Ltd., for peptide synthesis) was added, and the contents were thoroughly mixed. 14 mL of trifluoroacetic acid was further added to the mixture, followed by dilution in a measuring flask to 25 mL with distilled water. The resultant was used as a sample for HPLC measurement. The sample for HPLC measurement was subjected to HPLC measurement under the following conditions, and a decomposition ratio after the pancreatic juice treatment was determined on the basis of the following equation. The distilled water to be used for preparation was subjected to HPLC measurement under the same conditions as the sample solution, and the result was subtracted from each chromatogram to perform baseline correction.

Decomposition ratio in pancreatic juice treatment (%)=(peak area of self-assembling peptide in sample immersed in PBS-peak area of self-assembling peptide in sample immersed in simulated pancreatic juice)÷peak area of self-assembling peptide in sample immersed in PBS×100

<HPLC Measurement Conditions>

·Device 1 (used for analysis of self-assembling peptide gels 1 to 4): manufactured by Waters, M515 Pump, In-Line Degasser AF, 717 Autosampler, 996 Photodiode Array Detector, 2410 Refractive Index Detector, column heater
·Device 2 (used for analysis of self-assembling peptide gels 5 to 7): manufactured by Waters, Waters e2695 Separations Module, Waters 2998 Photodiode Array Detector, Waters SMH Column Heater, Empower 3 Feature Release 3 Hotfix 1, column heater: JASCO 860-CO
·Column: manufactured by YMC, YMC-Triart C18 3 mm×250 mm, φ3 μm
·Column heater preset temperature: 67°C
·Detection wavelength: 205 nm
·Analysis time: 60 minutes (self-assembling peptide gels 3 and 5 to 7) or 110 minutes (self-assembling peptide gels 1, 2, and 4)
·Mobile phase:

Solution A distilled water: 600 mL, acetonitrile: 200 mL, trifluoroacetic acid: 0.8 mL
Solution B distilled water: 250 mL, acetonitrile: 250 mL, trifluoroacetic acid: 0.5 mL

Table 2

| Composition of Mobile Phase | | |
|---|---|---|
| Time [min] | Solution A [%] | Solution B [%] |
| 0.0 | 95 | 5 |
| 15.00 | 95 | 5 |
| 30.00 | 75 | 25 |
| 40.00 | 75 | 25 |
| 50.00 | 0 | 100 |
| 60.00 | 0 | 100 |
| 61.00 | 95 | 5 |

Table 3

| Composition of Mobile Phase | | |
|---|---|---|
| Time [min] | Solution A [%] | Solution B [%] |
| 0.0 | 100 | 0 |
| 75.00 | 60 | 40 |
| 105.00 | 0 | 100 |
| 110.00 | 0 | 100 |

(continued)

| Composition of Mobile Phase | | |
|---|---|---|
| Time [min] | Solution A [%] | Solution B [%] |
| 110.01 | 100 | 0 |
| ·Flow rate: 0.4 mL/min<br>·Injection volume: 60 μL | | |

<<Adhesion Persistence Ratio>>

A collagen sheet (manufactured by Nippi Collagen Industries, Ltd., product number: "40322223") was bored with a trephine (8 mm), and immersed in physiological saline for 30 minutes or more. Onto the collagen sheet that had been removed from the physiological saline and lightly strained of water, 10 μL of a sample (self-assembling peptide gel) was applied, and the weight (A) of the collagen sheet after the application was measured. Then, the surface of the collagen sheet opposite to the surface to which the sample had been applied was lightly wetted with physiological saline containing 0.1% of a product named "Pancreatin Maruishi", and the resultant was attached to a 6-well plate (manufactured by Corning, product name: "Cell Culture 6-well Multiwell Plate") so that the sample-applied surface was an upper surface. The plate was centrifuged with a plate centrifuge (manufactured by Kubota Corporation, product name: "PlateSpin") under the conditions of 800 rpm and 20 seconds. After the centrifugation, the collagen sheet was detached from the plate, the weight (B) thereof was measured, and an adhesion persistence ratio calculated on the basis of the following equation was determined. With regard to each sample, the measurement and the calculation of the adhesion persistence ratio were performed for N=8, and the average value thereof was adopted as the adhesion persistence ratio of each sample.

$$\texttt{Adhesion persistence ratio (\%)=(B)/(A)×100}$$

[Preparation Example 1: Self-assembling Peptide Gel 1]

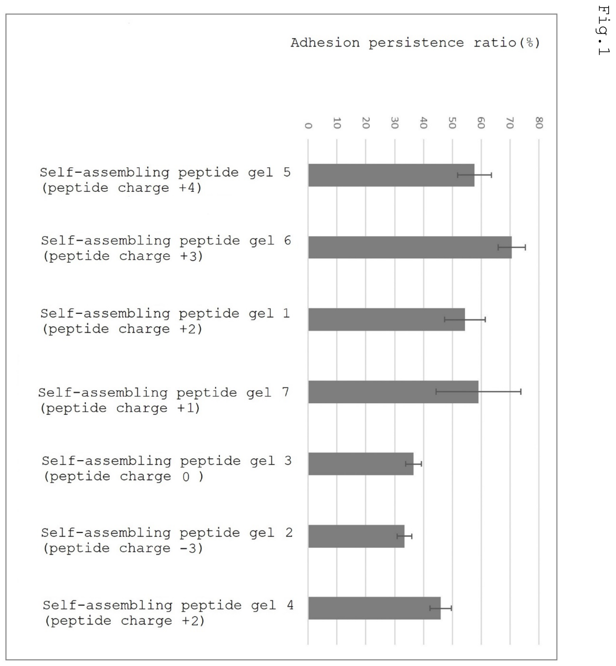

A high-pressure steam-sterilized self-assembling peptide gel (pH 5.9) containing 1.5 w/w% of a self-assembling peptide (SEQ ID NO: 1: Ac-RLDLRLALRLDLR-CONH$_2$), a tonicity agent, a pH adjuster, and water was used as a self-assembling peptide gel 1. The decomposition ratio of the self-assembling peptide gel 1 after the pancreatic juice treatment was 8.8%, and its viscosity was 35 Pa·s or more (above the measurement range). In addition, as shown in FIG. 1, the adhesion persistence ratio was 54.4%.

[Preparation Example 2: Self-assembling Peptide Gel 2]

1.5 w/w% of a self-assembling peptide (SEQ ID NO: 14: Ac-SAEASAEASAEAKAEA-CONH$_2$), a tonicity agent, a pH adjuster, and water were mixed to provide a self-assembling peptide gel 2 (pH 5.8). The decomposition ratio of the self-assembling peptide gel 2 after the pancreatic juice treatment was 10% or less. In addition, as shown in FIG. **1,** the adhesion persistence ratio was 33.4%.

[Preparation Example 3: Self-assembling Peptide Gel 3]

A self-assembling peptide gel (pH 2.2) containing 1.5 w/w% of a self-assembling peptide (SEQ ID NO: 15: Ac-RADARADARADARADA-CONH$_2$) and water was used as a self-assembling peptide gel 3. The decomposition ratio of the self-assembling peptide gel 3 after the pancreatic juice treatment was 10% or less, and its viscosity was 11.0 Pa·s. In addition, as shown in FIG. **1,** the adhesion persistence ratio was 36.5%.

[Preparation Example 4: Self-assembling Peptide Gel 4]

0.8 w/w% of a self-assembling peptide (SEQ ID NO: 1: Ac-RLDLRLALRLDLR-CONH$_2$), a tonicity agent, a pH adjuster, and water were mixed to provide a self-assembling peptide gel 4 (pH 6.0). The decomposition ratio of the self-assembling peptide gel 4 after the pancreatic juice treatment was 28%, and its viscosity was 11.2 Pa·s. In addition, as shown in FIG. **1,** the adhesion persistence ratio was 45.9%.

[Preparation Example 5: Self-assembling Peptide Gel 5]

1.5 w/w% of a self-assembling peptide (SEQ ID NO: 16: Ac-RASARASARASARASA-CONH$_2$), a tonicity agent, a pH adjuster, and water were mixed to provide a self-assembling peptide gel 5 (pH 6.8). The decomposition ratio of the self-assembling peptide gel 5 after the pancreatic juice treatment was 30.4%. In addition, as shown in FIG. **1,** the adhesion persistence ratio was 57.7%.

[Preparation Example 6: Self-assembling Peptide Gel 6]

1.5 w/w% of a self-assembling peptide (SEQ ID NO: 17: Ac-RASARASARASARADA-CONH$_2$), a tonicity agent, a pH adjuster, and water were mixed to provide a self-assembling peptide gel 6 (pH 6.2). The decomposition ratio of the self-assembling peptide gel 6 after the pancreatic juice treatment was 5.7%. In addition, as shown in FIG. **1,** the adhesion persistence ratio was 70.6%.

[Preparation Example 7: Self-assembling Peptide Gel 7]

1.5 w/w% of a self-assembling peptide (SEQ ID NO: 18: Ac-RASARADARADARADA-CONH$_2$), a tonicity agent, a pH adjuster, and water were mixed to provide a self-assembling peptide gel 7 (pH 7.7). The decomposition ratio of the self-assembling peptide gel 7 after the pancreatic juice treatment was 2.1%. In addition, as shown in FIG. **1,** the adhesion persistence ratio was 59.0%.

[Test Example 1]

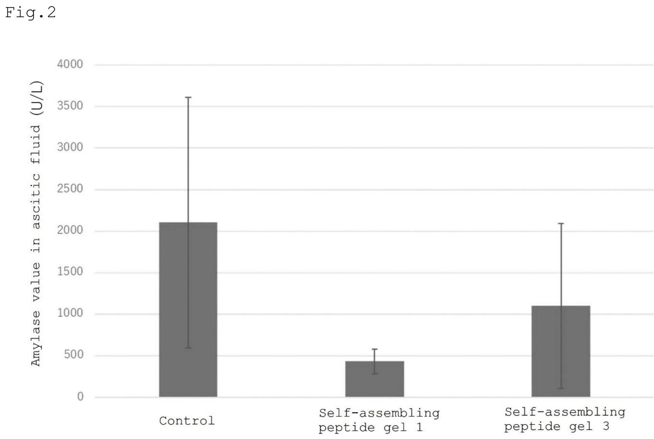

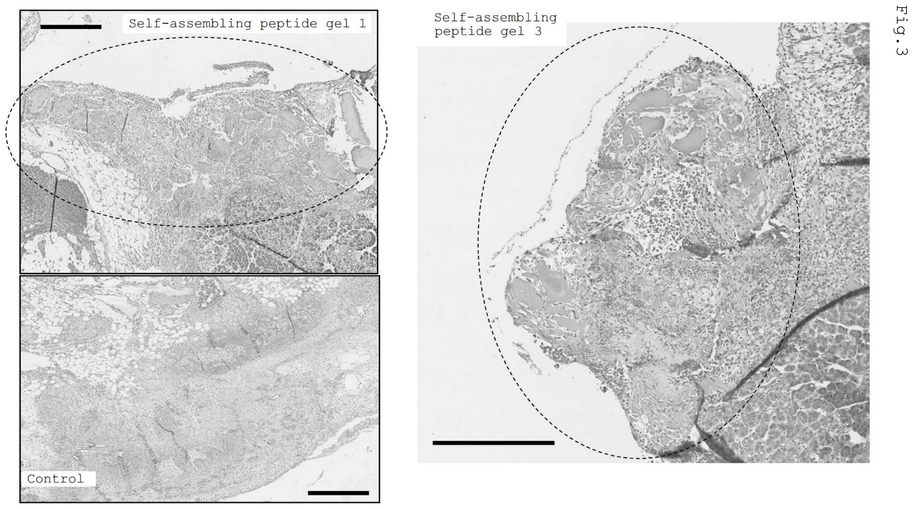

The abdomen of SD rats (8 weeks old) was opened under deep anesthesia, and part of the pancreas was resected. The self-assembling peptide gel 1 or the self-assembling peptide gel 3 was applied at a thickness of 0.1 mm or more so as to cover the resection surface, and the abdomen was closed. In addition, a rat whose abdomen was closed without the application of anything to the resection surface was used as a control. After 3 days from the operation, the abdomen was opened again to collect an ascitic fluid, and the rats were euthanized (N=9). After that, an amylase value (Amy) in the ascitic fluid was measured. The results are shown in FIG. **2** (mean$\pm$SD). In addition, microscopic observation photographs of the resection sites on the pancreas after 3 days from the operation are shown in FIG. **3.**
As apparent from FIG. **2,** the self-assembling peptide gel 1, which has an adhesion persistence ratio with respect to the collagen sheet equal to or higher than a predetermined value, and has a decomposition ratio equal to or lower than a predetermined value in the pancreatic juice treatment, can more suitably prevent a leak of pancreatic juice than the self-assembling peptide gel 3.
In addition, as shown in FIG. **3,** the surface of the pancreas having the self-assembling peptide gel 3 applied thereto has a remarkably small amount of the self-assembling peptide gel persisting thereon as compared to the surface of the pancreas having the self-assembling peptide gel 1 applied thereto. Thus, it is found that, due to a difference in adhesion persistence ratio of the self-assembling peptide gel to the digestive organ, a difference in digestive fluid leak preventative effect occurred. In addition, in consideration of FIG. **1,** it is suggested that, due to the charge of the self-assembling peptide, a difference in adhesive force with the surface of the organ occurs.

Industrial Applicability

The digestive fluid leak preventative material and the organ protective material of the present invention can be suitably used in the field of medical treatment.

**Claims**

**1.** A self-assembling peptide gel for use in preventing a leak of digestive fluid,

wherein the self-assembling peptide gel comprises:

a self-assembling peptide; and
water,

the self-assembling peptide gel has an adhesion persistence ratio with respect to a collagen sheet of at least 40% and a decomposition ratio of 35% or less in pancreatic juice treatment at 37°C for 7 days, and

the self-assembling peptide gel is positively charged as a whole.

2. The self-assembling peptide gel for use in preventing a leak of digestive fluid according to claim 1, wherein the self-assembling peptide includes a self-assembling peptide having a positive charge at a physiological pH.

3. The self-assembling peptide gel for use in preventing a leak of digestive fluid according to claim 2, wherein the self-assembling peptide includes a self-assembling peptide having a charge of from +1 to +4 at the physiological pH.

4. The self-assembling peptide gel for use in preventing a leak of digestive fluid according to any one of claims 1 to 3, wherein the self-assembling peptide gel has a viscosity of 3.0 Pa·s or more, which is measured under a temperature condition of 25°C using a rotational viscometer at a rotational speed of 0.5 rpm.

5. The self-assembling peptide gel for use in preventing a leak of digestive fluid according to any one of claims 1 to 4, wherein the self-assembling peptide includes a self-assembling peptide containing the following amino acid sequence (A):

   Amino acid sequence (A): $a_1b_1c_1b_2a_2b_3db_4a_3b_5c_2b_6a_4$

   in the amino acid sequence,

   $a_1$ to $a_4$ each represent a basic amino acid residue;
   $b_1$ to $b_6$ each represent an uncharged polar amino acid residue and/or a hydrophobic amino acid residue, provided that at least five thereof represent hydrophobic amino acid residues;
   $c_1$ and $c_2$ each represent an acidic amino acid residue; and
   "d" represents a hydrophobic amino acid residue or an uncharged polar amino acid residue.

6. The self-assembling peptide gel for use in preventing a leak of digestive fluid according to any one of claims 1 to 4, wherein the self-assembling peptide includes at least one kind selected from self-assembling peptides set forth in SEQ ID NOS: 1 to 13 and 16 to 18.

7. The self-assembling peptide gel for use in preventing a leak of digestive fluid according to any one of claims 1 to 6, wherein the digestive fluid is pancreatic juice.

8. A self-assembling peptide gel for use in protecting an organ against digestion by a digestive fluid,

   wherein the self-assembling peptide gel comprises:

      a self-assembling peptide; and
      water,

   the self-assembling peptide gel has an adhesion persistence ratio with respect to a collagen sheet of at least 40% and a decomposition ratio of 35% or less in pancreatic juice treatment at 37°C for 7 days, and
   the self-assembling peptide gel is positively charged as a whole.

9. The self-assembling peptide gel for use in protecting an organ against digestion by a digestive fluid according to claim 8, wherein the self-assembling peptide includes a self-assembling peptide having a positive charge at a physiological pH.

10. The self-assembling peptide gel for use in protecting an organ against digestion by a digestive fluid according to claim 9, wherein the self-assembling peptide includes a self-assembling peptide having a charge of from +1 to +4 at the physiological pH.

11. The self-assembling peptide gel for use in protecting an organ against digestion by a digestive fluid according to any one of claims 8 to 10, wherein the self-assembling peptide gel has a viscosity of 3.0 Pa·s or more, which is measured under a temperature condition of 25°C using a rotational viscometer at a rotational speed of 0.5 rpm.

12. The self-assembling peptide gel for use in protecting an organ against digestion by a digestive fluid according to any one of claims 8 to 11, wherein the self-assembling peptide includes a self-assembling peptide containing the following amino acid sequence (A):

Amino acid sequence (A): $a_1b_1c_1b_2a_2b_3db_4a_3b_5c_2b_6a_4$

in the amino acid sequence,

$a_1$ to $a_4$ each represent a basic amino acid residue;
$b_1$ to $b_6$ each represent an uncharged polar amino acid residue and/or a hydrophobic amino acid residue, provided that at least five thereof represent hydrophobic amino acid residues;
$c_1$ and $c_2$ each represent an acidic amino acid residue; and
"d" represents a hydrophobic amino acid residue or an uncharged polar amino acid residue.

13. The self-assembling peptide gel for use in protecting an organ against digestion by a digestive fluid according to any one of claims 8 to 11, wherein the self-assembling peptide includes at least one kind selected from self-assembling peptides set forth in SEQ ID NOS: 1 to 13 and 16 to 18.

14. The self-assembling peptide gel for use in protecting an organ against digestion by a digestive fluid according to any one of claims 8 to 13, wherein the digestive fluid is pancreatic juice.

Fig.1

Fig.2

Fig.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019508175 A
- WO 2007000979 A1